# FASCICULE DE BREVET EUROPEEN

(11) **EP 2 818 126 B1**
(45) Date de publication et mention de la délivrance du brevet: **09.08.2017**
(21) Numéro de dépôt: 14171950.0
(22) Date de dépôt: 11.06.2014
(51) Int. Cl.: A61B 17/16

(54) **Porte-fraise à poignée**
Fräserhalter mit Griff
Reamer holder with handle

(30) Priorité: 26.06.2013 FR 1301495
(43) Date de publication de la demande: 31.12.2014
(73) Titulaire: XNOV IP, 1882 Luxembourg (LU)
(72) Inventeur: Biegun, Jean-François, 2900 Prrentruy (CH)
(74) Mandataire: Eidelsberg, Olivier Nathan

(56) Documents cités:
- US-A1- 2004 122 460
- US-A1- 2005 216 022
- US-A1- 2006 041 268
- US-A1- 2007 073 302

## Description

La présente invention se rapporte à un dispositif formant porte-outil pour couper, râper ou enlever de l'os, notamment un porte-râpe ou un porte-fraise, en particulier pour râper de l'os d'une hanche.

Le porte-outil, notamment le porte-fraise, réalise l'interface entre l'outil, notamment la fraise, et un moteur d'entraînement, notamment électrique, destiné à entraîner en rotation l'outil, notamment la fraise, pour râper de l'os.

On connaît déjà des portes-fraise de ce genre. Ils sont constitués d'un corps creux de forme oblongue, sensiblement cylindrique, définissant un canal interne dans lequel passe une tige de transmission destinée à réaliser la transmission entre d'une part la fraise disposée à une extrémité distale du porte fraise et d'autre part un axe de rotation du moteur à une extrémité proximale opposée à l'extrémité distale. Le dispositif comporte en outre un élément formant poignée destiné à la préhension du porte-outil par le chirurgien.

Ces portes-fraise de l'art antérieur doivent être particulièrement robustes pour supporter les contraintes liées à la rotation du moteur et à l'action de la fraise lors de la râpe. Il en résulte que ces dispositifs non seulement sont de structures compliquées et en outre s'usent rapidement.

Le document US 2005/0216022 A1 divulgue un porte-outil selon le préambule de la revendication 1. La présente invention vise à surmonter les inconvénients de l'art antérieur en proposant un porte-outil, notamment un porte-fraise, qui d'une part soit de structure simple, notamment plus simple que celles des porte fraise de l'art antérieur, et qui d'autre part a une plus grande longévité en résistant mieux aux efforts et contraintes liés à l'action de la râpe et à l'entraînement du moteur.

Suivant l'invention, un porte-outil, notamment un porte-fraise est tel que défini à la revendication 1, des perfectionnements et modes de réalisation préférés étant définis aux sous revendications 2 à 11.

En prévoyant ainsi un corps en deux parties et notamment une partie auxiliaire portant l'élément de préhension, on facilite d'une part l'utilisation du porte outil et d'autre part sa résistance globale aux contraintes liées à l'action de la râpe et à l'entraînement du moteur, l'ensemble étant moins rigide les contraintes mieux amorties. En outre, on peut plus facilement réaliser un blocage en translation dans la direction longitudinale de déplacement de l'axe de transmission au niveau de l'interface transmission-axe du moteur, de sorte que la transmission reste bien immobile dans la direction longitudinale et on évite ainsi des déplacements longitudinaux sources de contrainte élevée sur le reste du dispositif, et notamment sur le corps principal, ce qui jusqu'à maintenant dans l'art antérieur entraînait des usures prématurées du porte outil. En outre, comme la partie principale du corps subit moins de contrainte, on peut réaliser le porte-outil avec une structure plus simple. En particulier, si on le souhaite, on peut se passer de prévoir des éléments dans la partie principale du corps destinés à amortir les effets liés au déplacement en translation (qui n'existe plus ou qui en tout cas est fortement diminué) de la transmission au sein de la partie principale du corps. Il en résulte un porte outil de structure plus simple, plus légère et de plus grande longévité.

Suivant un perfectionnement qui en soit constitue également une invention indépendante de l'invention décrite précédemment mais qui peut être mis en oeuvre en combinaison avec cette dernière de manière avantageuse, la transmission comporte au moins deux tiges reliées par une liaison cardan constituée d'une fourchette issue de la tige, de deux demi-fourchettes issues de la tige et d'une demi-goupille et d'un croisillon monoblocs.

On décrit maintenant, à titre d'exemple, un mode de réalisation de l'invention en se reportant aux dessins, dans lesquels :
- la figure 1: est une vue de côté de trois éléments constitutifs principaux d'un dispositif porte-outil suivant l'invention ;
- la figure 2: est une vue éclatée en perspective de la partie auxiliaire comportant l'élément de préhension du dispositif de la figure 1, la pièce intermédiaire de liaison entre la transmission et l'axe du moteur et l'élément amortisseur étant représentés à l'état sortis de la partie auxiliaire, alors qu'à la figure 1 ils sont représentés à l'état introduits;
- la figure 3: est une vue en coupe longitudinale d'une partie du porte outil de la figure 1, au niveau de la partie auxiliaire comportant l'élément de préhension ;
- la figure 4: est une vue en perspective du porte outil de la figure 1 à l'état assemblé avec cependant le tube 21 et un élément raccord entre la partie auxiliaire portant l'élément de préhension et la partie principale de corps qui ont été omis (ces éléments sont en revanche représentés à la figure 1) ;
- la figure 5: est une vue en perspective de la partie d'extrémité proximale de la partie principale de corps du porte outil des figures précédentes ;
- la figure 6: est une vue en perspective du côté distal de la partie auxiliaire portant l'élément de préhension des figures 1 à 4 ;
- la figure 7: est une vue en perspective plus en détail de la partie proximale de la transmission du porte outil des figure précédentes ;
- la figure 8: est une vue en perspective de la partie distale de la transmission du porte outil ensemble de la figure 7 ; et
- la figure 9: est une vue à plus grande échelle et éclatée d'une partie de la figure 8.

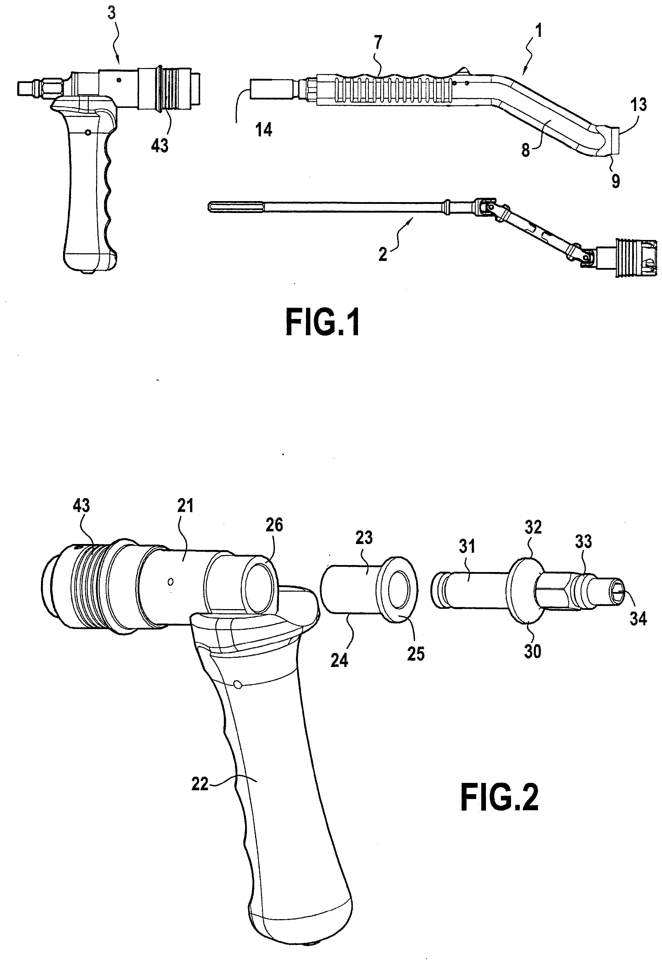

A la figure 1, il est représenté trois parties principales d'un porte-râpe suivant l'invention, respectivement une partie 1 principale de corps, un élément 2 de transmission et une partie 3 auxiliaire portant un élément 22 de préhension. Les deux parties 1 et 2 principale et auxiliaire, respectivement distale et proximale, forment de manière générale le corps du dispositif qui reçoit en son sein la transmission 2.

Le partie 1 principale est un élément oblong de forme sensiblement cylindrique de section transversale sensiblement carrée et formé d'une paroi 4 de dessus et de deux parois latérales, les trois parois de dessus et latérales définissant entre elle un canal 5 interne vers le bas et destiné à recevoir en son sein la transmission 2. La partie 1 principale comporte en outre une ouverture 13 latérale distale et une ouverture 14 distale.

A son extrémité proximale, la partie principale est terminée par un tronçon 6 proximal de section sensiblement circulaire de plus petite dimension que le reste du corps principal. Le canal 5 se poursuit à l'intérieur du tronçon 6 jusqu'à l'ouverture latérale 13 proximale, puis dans la partie 3 auxiliaire jusqu' à une ouverture proximale de cette dernière, délimitée par un bord 26 proximal.

En outre, la partie 1 principale est constituée de trois tronçons 7, 8, 9, les deux tronçons 7, 9 étant parallèles entre eux tandis que le tronçon 8 intermédiaire est incliné par rapport aux deux tronçons 7 et 9 d'extrémité proximale et distale, notamment d'un angle d'environ 45°. Cette inclinaison, classique dans le domaine des porte-fraise, permet de décaler l'axe de la fraise de la zone encombrée par les tissus mous de la jambe de la personne dans laquelle on effectue la râpe, ce qui simplifie l'action du chirurgien. Cependant, la présente invention n'est pas limitée à cette forme inclinée et on pourrait également prévoir, sans sortir du cadre de la présente invention, un corps principal qui soit cylindrique rectiligne ou une forme inclinée avec un angle d'inclinaison différent.

L'élément 2 de transmission comporte quatre parties 10, 11, 12, 20 articulées en succession les une aux autres, à savoir trois parties 10, 11 , 12 en forme de tige articulées par des systèmes à cardan et une partie 20 de raccordement destinée à assurer la connexion à la fraise.

La partie 20 de raccordement comporte un élément en forme de tube fileté à l'extérieur et est bien connu dans le domaine, de sorte qu'il n'est pas décrit plus avant.

La tige 10 proximale comporte un tronçon distal 15 dont la section est en forme à six pans, tandis que le reste de la tige est à section transversale circulaire.

Les trois tiges articulées de la transmission 2, en utilisation, s'étendent dans le canal 5, tandis que la partie 12 de raccordement à la fraise fait saillie hors du corps par l'ouverture 14 distale. L'extrémité proximale de la tige 10 fait saillie de l'ouverture 13 proximale latérale du corps 1.

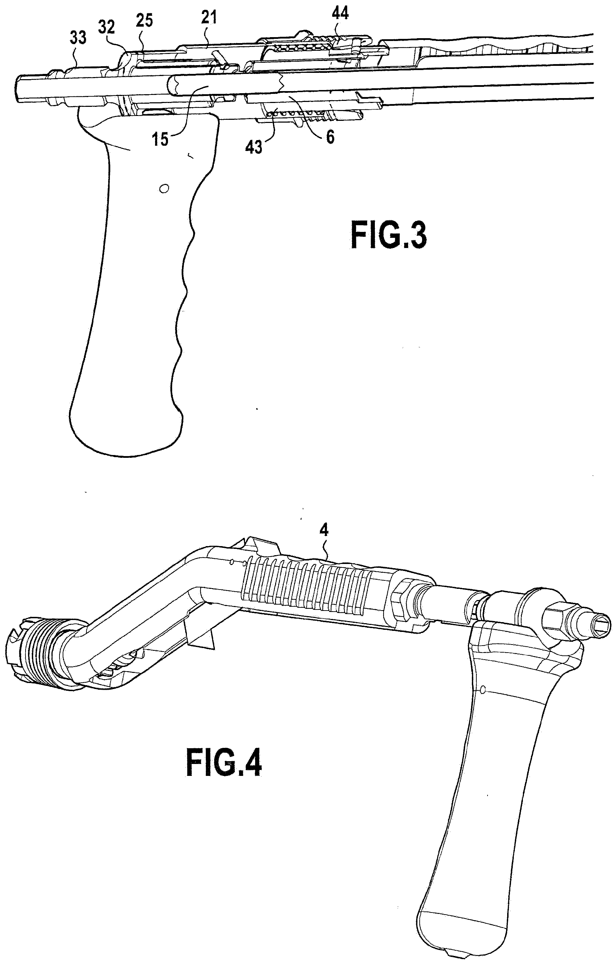

La partie 3 auxiliaire portant l'élément de préhension est constitué d'un élément 21 en forme de tube destiné à recevoir en son sein une partie au moins du tronçon 15 d'extrémité proximale de la tige 10. Autour du tube 21, il est monté une bague 23 de raccord (représentée aux figures 1 et 2, mais pas à la figure 4) destinée à assurer la liaison entre la partie auxiliaire 3 et la partie 1 principale du corps du porte-outil. De l'extrémité proximale du tube 21, un barreau fait saillie latéralement. Ce barreau de forme quelconque, mais allongée de préférence et de forme cylindrique circulaire notamment, n'est pas visible aux figures car il est recouvert d'un surmoulage 22 ayant une forme s'adaptant à la forme de la main pour permettre la préhension du porte outil.

Le tube 21 de la partie 3 reçoit en son sein un élément 23 en matière élastomère comportant un élément en forme de tube 24 et une bague 25 faisant saillie latéralement du tube 24 pour former un épaulement destiné à venir buter contre le bord 26 de l'ouverture proximale du tube 21. Cette élément 23 élastomère a pour fonction d'amortir les chocs et vibrations liés à la liaison entre le moteur électrique et la transmission 2, liaison qui s'effectue au sein du tube 21 par l'intermédiaire d'une pièce 30 intermédiaire entre l'axe du moteur (non représenté et le tronçon 15 de la tige 10 proximale de la transmission 2.

La pièce 30 intermédiaire comporte d'une part un tronçon 31 en forme de tube destiné à être inséré dans l'élément 21 avec interposition du tube 24 élastomère. Le tronçon distal 21 en forme de tube définit en son sein un passage pour la transmission. Sa section transversale est de forme complémentaire de celle du tronçon 15 (dans le mode de réalisation ici représenté, la forme est à 6 pans, mais on pourrait prévoir d'autres formes) pour assurer une solidarisation en rotation de la tige 10 de la transmission et de la pièce 30 intermédiaire.

Une bague 32 fait saillie latéralement de la pièce 30 intermédiaire et est destinée à buter contre le bord 26 avec interposition de la bague 25 élastomère pour ainsi empêcher un mouvement dans la direction longitudinal de la transmission. La pièce 30 est terminée du côté proximal par un tronçon 33 sensiblement de forme cylindrique destiné à la liaison avec l'axe du moteur (non représenté) En particulier le tronçon 33 comporte un trou 34 borgne de réception de l'axe du moteur ayant une section transversale en forme à six pans.

Les dimensions du tube 23 et du tube 31 sont telles que ce dernier s'adapte à force dans le premier, qui lui-même s'adapte en force dans le tube 21 en passant par l'ouverture 19 pour ainsi assurer une fixation de la pièce 30 intermédiaire à l'élément 21 en forme de tube de la pièce de préhension.

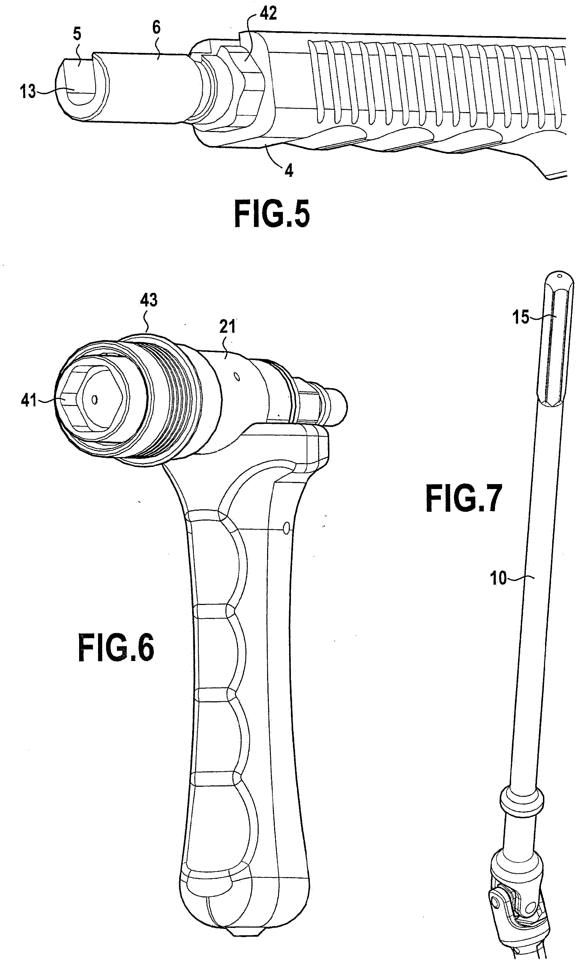

A la figure 6, il est représente l'autre extrémité que l'on ne voit pas suivant la figure 2. L'élément 21 en forme de tube est prolongé par un élément 40 tubulaire dont l'ouverture 41 distale est de forme complémentaire du tronçon 42 à 6 pans formé sur le corps principal, à la suite distalement du tronçon 6 de plus petit diamètre.

Ainsi, le tronçon 6 cylindrique circulaire pénètre, en même temps que la tige 10 de la transmission dans l'élément 40 tubulaire de prolongation jusqu'à ce que le tronçon 42 viennent s'adapter à l'ouverture 41 pour bloquer en rotation l'élément de préhension et le corps principal. La fixation ensemble des deux pièces est ensuite assuré par deux bagues 43 et 44 respectivement filetée et taraudée solidaires respectivement de l'élément de préhension et du corps principal. La bague 43 est représentée aux dessins aux figures 1, 2, 3 et 6 tandis que la bague 44 n'est représentée qu'à la figure 3. En utilisation, et notamment comme représenté à la figure 3 vue en coupe transversale, les tube 24 et 31 sont reçues dans le tube 21, l'extrémité 15 proximale de la tige 10 pénétrant dans le tube 31 de la pièce 30 intermédiaire en étant à complémentarité de forme avec la section transversale intérieure de cet élément en forme de tube 31, de sorte que l'élément en forme de tube est solidarisé en rotation avec la transmission 2. Dans le même temps, la bague 32 vient presser contre la bague 25 et contre le bord 26 proximal de l'ouverture du tube 21 et l'axe du moteur vient engrener dans le trou borgne 34, pour ainsi solidariser en rotation l'axe du moteur avec la transmission 2.

Suivant l'invention, le moteur, compte tenu de la butée 32 et de la bague 25, est empêché d'avoir une action dans la direction longitudinale vis-à-vis de l'axe de transmission 2, et par conséquent l'axe de transmission 2 est beaucoup plus stable dans cette direction longitudinale. Il en résulte que la transmission 2 reste bien plus stable dans le corps 1 principal et n'a pas tendance à en sortir, de sorte qu'il n'est pas nécessaire, comme c'était le cas dans l'art antérieur, de prévoir de nombreux paliers de blocage et/ou fixations des différentes tiges de la transmission dans le corps 1, 3. En outre, le corps peut être ouvert d'un côté au moins sur une partie, notamment le long de la partie 1 principale du corps, ici vers le bas, sans que cela ne pose de difficulté, alors que dans l'art antérieur une telle ouverture aurait présenté des inconvénients. Il en résulte que la structure du corps est plus légère et plus simple.

En outre, la coopération de l'ouverture 41 et du tronçon 42 permet un réglage au choix du chirurgien parmi plusieurs orientations possibles du tronçon 42 par rapport à l'élément de préhension, chaque orientation correspondant à une position relative correspondante de la forme à 6 pans du tronçon dans l'ouverture ayant une section également à 6 pans correspondantes.

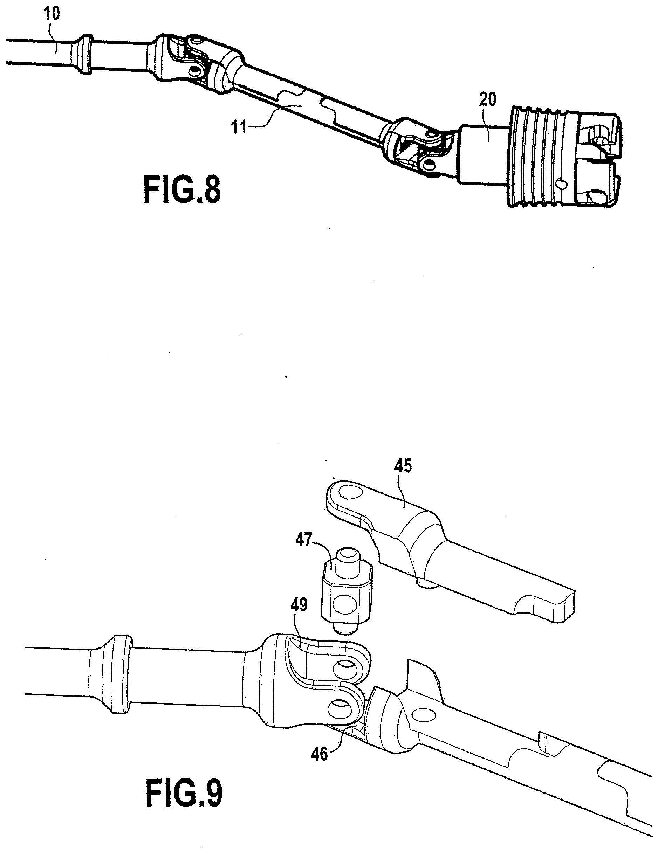

Aux figures 8 et 9, il est représenté un mode de réalisation particulier de la transmission suivant l'invention qui présente un aspect inventif indépendamment de celui décrit ci dessus.

A la figure 8, il est représenté suivant une vue en perspective éclatée la transmission 2. Entre les deux tiges 10 et 11, la liaison est en cardan. Ce dernier est constitué d'une fourchette 44 issue de la tige 10, de deux demi-fourchettes 45, 46 issues de la tige 11 et d'une demi-goupille 47 monobloc. La demi-goupille et le croisillon sont monoblocs. Cela augmente considérablement la résistance mécanique de l'ensemble et permet de réduire le diamètre des joints. La zone de friction goupille/fourchette est plus éloignée du centre de rotation, ce qui diminue les efforts et réduit le jeu et donc l'usure, ce qui permet l'assemble de l'une des deux fourchettes constituées en deux coquilles. Cet assemblage en deux coquilles permet d'assembler le système qui n'est plus fermé comme dans l'art antérieur par des demi-goupilles, mais par une goupille complète.

Entre la tige 11 et l'élément 20 de raccordement, il y a également une liaison cardan, la fourchette issue de la tige 11 étant également constituée de deux demi fourchettes, identiques à celles de son autre extrémité.

En prévoyant que le système de couplage au moteur et le système de réglage à la poignée soient couplés sur le même verrou, on obtient un dispositif beaucoup plus simple d'utilisation et/ou un système plus sûr. En effet, maintenant lorsque l'on souhaite régler la poignée, il suffit uniquement de déverrouiller le verrou commun, ce qui garantit dans le même temps une déconnexion du moteur, puis une fois la poignée réglée le reverrouillage du verrou assurant à la fois le verrouillage de la poignée dans sa bonne position et la reconnexion du moteur. En s'assurant ainsi que l'on n'a plus à régler la poignée alors même que le moteur est encore en prise. Dans ce cas là, il est également nécessaire de déconnecter le moteur, ce qui est source de complexité et de danger.

## Revendications

1. Porte-outil, notamment un porte-fraise, qui comprend un corps de forme sensiblement cylindrique oblongue définissant un canal (5) dans lequel passe une transmission (2) destinée à transmettre à l'outil, notamment la fraise, un mouvement issu de l'axe d'un moteur d'entraînement agissant sur une extrémité proximale de la transmission, et un élément de préhension pour la préhension du porte-outil fixé au corps, **caractérisé en ce que** le corps est réalisé en au moins deux parties distinctes, une partie (1) principale distale et une partie (3) auxiliaire proximale, le canal ayant un tronçon respectif défini par chaque partie, l'élément de préhension étant fixé à la partie auxiliaire et l'extrémité proximale de la transmission se trouvant dans le tronçon du canal défini par la partie auxiliaire.

2. Porte outil suivant la revendication 1, **caractérisé en ce que** les deux parties distinctes principale et auxiliaire sont disposées l'une à la suite de l'autre.

3. Porte outil suivant la revendication 2, **caractérisé en ce que** les deux parties principale et auxiliaire sont fixés l'une à l'autre de manière amovible par des moyens de fixation amovible.

4. Porte outil suivant la revendication 3, **caractérisé en ce que** les moyens de fixation amovible comportent deux bagues solidaires respectivement des deux parties principale et auxiliaire.

5. Porte-outil suivant l'une des revendications 1 à 4, **caractérisé en ce qu'**il est prévu des moyens de réglage de l'orientation relative en rotation de la partie auxiliaire par rapport à la partie principale, notamment par la coopération d'un tronçon en forme à 6 pans du corps principal avec une ouverture à section transversale à 6 pans de la partie (3) auxiliaire.

6. Porte-outil suivant l'une des revendications 1 à 5, **caractérisé en ce qu'**il est prévu une pièce (30) intermédiaire disposée entre la transmission (2) et l'axe du moteur d'entraînement, la pièce intermédiaire comportant des moyens pour permettre de solidariser en rotation cette pièce intermédiaire à la transmission et à l'axe du moteur, la pièce intermédiaire comportant des moyens (32) formant butée destinés à bloquer en translation le long de l'axe longitudinal de transmission son déplacement par butée, notamment contre la partie (3) auxiliaire.

7. Porte-outil suivant la revendication 6, **caractérisé en ce que** la pièce intermédiaire est reçue en partie dans la partie auxiliaire pour y être solidarisée en rotation avec la transmission.

8. Porte-outil suivant l'une des revendications 1 à 7, **caractérisé en ce que** la partie (3) auxiliaire reçoit en son sein un élément (23) en matière élastomère comportant un élément en forme de tube (24) et une bague (25) faisant saillie latéralement du tube (24) pour former un épaulement destiné à venir buter contre le bord (26) d'une ouverture proximale de la partie auxiliaire (3).

9. Porte-outil suivant les revendications 6 et 8, **caractérisé en ce que** la pièce (30) intermédiaire comporte un tronçon (31) en forme de tube destiné à être inséré dans l'élément (21) avec interposition du tube (24) élastomère.

10. Porte-outil suivant la revendication 6, **caractérisé en ce qu'**une bague (32) fait saillie latéralement de la pièce (30) intermédiaire et est destinée à buter contre le bord (26) de l'ouverture proximale d'un tube (21) de la partie (3) auxiliaire avec interposition de la bague (25) élastomère pour ainsi empêcher un mouvement dans la direction longitudinal de la transmission.

11. Porte-outil suivant l'une des revendications 6 à 10, **caractérisé en ce que** la pièce (30) est terminée du côté proximal par un tronçon (33) sensiblement de forme cylindrique destiné à la liaison avec l'axe du moteur.

12. Porte-outil suivant l'une des revendications précédentes, **caractérisé en ce que** la transmission (2) comporte au moins deux tiges (10, 11) reliées par une liaison cardan constituée d'une fourchette (44) issue de la tige (10), de deux demi-fourchettes (45, 46) issues de la tige (11) et d'une demi-goupille (47) et d'un croisillon monoblocs.

## Patentansprüche

1. Werkzeughalter, insbesondere Fräserhalter, der einen Körper mit einer im Wesentlichen zylindrischen, länglichen Form, der einen Kanal (5) definiert, in den eine Kraftübertragungseinrichtung (2) passt, die dazu bestimmt ist, auf das Werkzeug, insbesondere den Fräser, eine Bewegung zu übertragen, die von der Achse eines Antriebsmotors stammt, die auf ein proximales Ende der Kraftübertragungseinrichtung einwirkt, und eine Greifeinrichtung zum Greifen des am Körper befestigten Werkzeughalters umfasst, **dadurch gekennzeichnet, dass** der Körper aus mindestens zwei verschiedenen Teilen ausgeführt ist, einem distalen Hauptteil (1) und einem proximalen Hilfsteil (3), wobei der Kanal einen Abschnitt aufweist, der durch jedes Teil definiert wird, wobei die Greifeinrichtung am Hilfsteil befestigt ist und sich das proximale Ende der Kraftübertragungseinrichtung in dem Abschnitt des Kanals befindet, der durch das Hilfsteil definiert wird.

2. Werkzeughalter nach Anspruch 1, **dadurch gekennzeichnet, dass** die beiden verschiedenen Teile, Haupt- und Hilfsteil, hintereinander angeordnet sind.

3. Werkzeughalter nach Anspruch 2, **dadurch gekennzeichnet, dass** die beiden Teile, Hauptteil und Hilfsteil, mithilfe von lösbaren Befestigungsmitteln lösbar aneinander befestigt sind.

4. Werkzeughalter nach Anspruch 3, **dadurch gekennzeichnet, dass** die lösbaren Befestigungsmittel zwei Ringe umfassen, die jeweils mit den beiden Teilen, Hauptteil und Hilfsteil, fest verbunden sind.

5. Werkzeughalter nach einem der Ansprüche 1 bis 4, **dadurch gekennzeichnet, dass** Mittel zum Einstellen der relativen Drehausrichtung des Hilfsteils in Bezug auf das Hauptteil, insbesondere zum Zusammenwirken eines Abschnitts in Sechskantform des Hauptteils mit einer Öffnung mit Sechskantquerschnitt des Hilfsteils (3), vorgesehen sind.

6. Werkzeughalter nach einem der Ansprüche 1 bis 5, **dadurch gekennzeichnet, dass** ein Zwischenstück (30) vorgesehen ist, das zwischen der Kraftübertragungseinrichtung (2) und der Achse des Antriebsmotors angeordnet ist, wobei das Zwischenstück Mittel umfasst, die es ermöglichen, dieses Zwischenstück drehfest mit der Kraftübertragungseinrichtung und der Achse des Motors zu verbinden, wobei das Zwischenstück Mittel (32) umfasst, die einen Anschlag bilden, die dazu bestimmt sind, seine translationale Verschiebung entlang der Kraftübertragungslängsachse durch Anschlag, insbesondere gegen das Hilfsteil (3), zu blockieren.

7. Werkzeughalter nach Anspruch 6, **dadurch gekennzeichnet, dass** das Zwischenstück teilweise in das Hilfsteil aufgenommen ist, um dort drehfest mit der Kraftübertragungseinrichtung verbunden zu sein.

8. Werkzeughalter nach einem der Ansprüche 1 bis 7, **dadurch gekennzeichnet, dass** das Hilfsteil (3) in sich ein Element (23) aus Elastomermaterial aufnimmt, das ein Element in Rohrform (24) und einen Ring (25) umfasst, der von dem Rohr (24) seitlich vorsteht, um einen Absatz zu bilden, der dazu bestimmt ist, gegen den Rand (26) einer proximalen Öffnung des Hilfsteils (3) zur Anlage zu kommen.

9. Werkzeughalter nach den Ansprüchen 6 und 8, **dadurch gekennzeichnet, dass** das Zwischenstück (30) einen Abschnitt (31) in Rohrform umfasst, der dazu bestimmt ist, in das Element (21) eingeführt zu werden, wobei das Elastomerrohr (24) dazwischen eingefügt wird.

10. Werkzeughalter nach Anspruch 6, **dadurch gekennzeichnet, dass** ein Ring (32) seitlich von dem Zwischenstück (30) vorsteht und dazu bestimmt ist, gegen den Rand (26) der proximalen Öffnung eines Rohrs (21) des Hilfsteils (3) zur Anlage zu kommen, wobei dazwischen der Elastomerring (25) eingefügt wird, um dadurch eine Bewegung in der Kraftübertragungslängsrichtung zu verhindern.

11. Werkzeughalter nach einem der Ansprüche 6 bis 10, **dadurch gekennzeichnet, dass** das Stück (30) auf der proximalen Seite mit einem Abschnitt (33) endet, der im Wesentlichen zylinderförmig ist, der der Verbindung mit der Motorachse dient.

12. Werkzeughalter nach einem der vorhergehenden Ansprüche, **dadurch gekennzeichnet, dass** die Kraftübertragungseinrichtung (2) mindestens zwei Stangen (10, 11) umfasst, die über ein Kardangelenk verbunden sind, das aus einer Gabel (44) die aus der Stange (10) hervorgeht, aus zwei Gabelhälften (45, 46), die aus der Stange (11) hervorgehen, und aus einem Halbstift (47) und einem einstückigen Kreuzstück besteht.

## Claims

1. Tool holder, in particular a milling cutter holder, which comprises a substantially oblong cylindrical body defining a channel (5) into which a transmission (2) passes which is designed to transmit to the tool, in particular the milling cutter, a movement from the axis of a drive motor acting on a proximal end of the transmission, and a gripping element for gripping the tool holder fixed to the body, **characterised in that** the body is formed by at least two distinct parts, a distal main part (1) and a proximal auxiliary part (3), the channel having a respective section defined by each part, the gripping element being fixed to the auxiliary part and the proximal end of the transmission being located in the section of the channel designed by the auxiliary part.

2. Tool holder according to claim 1, **characterised in that** the two distinct parts, the main part and auxiliary part, are arranged consecutively.

3. Tool holder according to claim 2, **characterised in that** the two parts, the main part and the auxiliary part, are fixed to one another in a detachable manner by detachable securing means.

4. Tool holder according to claim 3, **characterised in that** the detachable securing means comprise two rings joined respectively to the two parts, the main part and auxiliary part.

5. Tool holder according to one of claims 1 to 4, **characterised in that** means are provided for adjusting the relative orientation in rotation of the auxiliary part to the main part, in particular by the cooperation of a section with 6 sides of the main body with an opening with a cross section of 6 sides of the auxiliary part (3).

6. Tool holder according to one of claims 1 to 5, **characterised in that** an intermediate part (30) is provided arranged between the transmission (2) and the axis of the drive motor, the intermediate part comprising means for enabling the rotatable connection of this intermediate part to the transmission and the axis of the motor, the intermediate part comprising means (32) forming an abutment designed to lock in translation in the longitudinal direction of displacement of the axis of transmission by abutment in particular against the auxiliary part (3).

7. Tool holder according to claim 6, **characterised in that** the intermediate part is received partly in the auxiliary part to be joined in rotation with the transmission.

8. Tool holder according to one of claims 1 to 7, **characterised in that** the auxiliary part (3) receives within it an element (23) made from an elastomer material comprising an element in the form of a tube (24) and a ring (25) projecting laterally from the tube (24) to form a shoulder designed to abut against the edge (26) of a proximal opening of the auxiliary part (3).

9. Tool holder according to claims 6 and 8, **characterised in that** the intermediate part (30) comprises a section (31) in the form of a tube designed to be inserted into the element (21) with the interposition of the elastomer tube (24).

10. Tool holder according to claim 6, **characterised in that** a ring (32) projects laterally from the intermediate part (30) and is designed to abut against the edge (26) of the proximal opening of a tube (21) of the auxiliary part (3) with the interposition of the elastomer ring (25) to thus prevent movement in the longitudinal direction of transmission.

11. Tool holder according to one of claims 6 to 10, **characterised in that** the part (30) is terminated at the proximal end by a section (33) with a substantially cylindrical form designed for connection with the axis of the motor.

12. Tool holder according to one of the preceding claims, **characterised in that** the transmission (2) comprises at least two rods (10, 11) connected by a cardan joint formed by a fork (44) coming from rod (10), two half-forks (45, 46) coming from rod (11) and a half-pin (47) and a single-piece brace.
